# EUROPEAN PATENT APPLICATION

(11) **EP 4 809 927 A1**
(43) Date of publication of application: **23.09.2026**
(21) Application number: 24900281.7
(22) Date of filing: 08.10.2024
(51) Int. Cl.: A61B 6/00

(54) **IMAGE PROCESSING METHOD, IMAGE PROCESSING DEVICE, AND IMAGE PROCESSING PROGRAM**

(30) Priority: 04.12.2023 JP 2023204616
(71) Applicant: Hamamatsu Photonics K.K., Hamamatsu-shi, Shizuoka 435-8558 (JP)
(72) Inventor: ONISHI Tatsuya, Hamamatsu-shi, Shizuoka 435-8558 (JP); TSUCHIYA Satoshi, Hamamatsu-shi, Shizuoka 435-8558 (JP)
(74) Representative: Hoffmann Eitle
(86) International application number: PCT/JP2024/035988
(87) International publication number: WO 2025/120984

(57) **Abstract**

The present invention addresses the problem of achieving stable noise reduction in an image having various luminance values. A control device 121 calculates a relationship between luminance values in an X-ray image and a statistical value of the luminance values, acquires an X-ray image to be processed, specifies an outlier pixel that deviates from a trend of output fluctuation indicated by the statistical value, derived on the basis of a luminance value of each pixel of the X-ray image and the relationship, using an evaluation value relating to fluctuation in the luminance value calculated for each pixel of the X-ray image, and processes the image using a filter that reduces the fluctuation in the luminance value for the outlier pixel in the X-ray image.

## Description

### Technical Field

The present invention relates to an image processing method, an image processing device, and an image processing program.

### Background Art

Conventionally, an imaging system that performs a noise reduction process on signals output from some of a plurality of pixels with respect to an image output from a detector having the plurality of pixels, has been used (see, for example, Patent Literature 1). This imaging system extracts some pixels by performing division of two images obtained in response to radiation radiated during two different periods.

### Citation List

### Patent Literature

[Patent Literature 1] Japanese Unexamined Patent Publication No. 2014-183475

### Summary of Invention

### Technical Problem

The noise reduction technique in the conventional imaging system as described above has tended to have difficulty in stably removing noise from pixels with large noise in an image having various luminance values. For this reason, there is a need to realize stable noise reduction in an image having various luminance values.

The present disclosure has been made in view of such a problem, and an object thereof is to provide an image processing method, an image processing device, and an image processing program that can realize stable noise reduction in an image having various luminance values.

### Solution to Problem

According to a first aspect of the embodiment, there is provided an image processing method including: a calculation step of calculating a relationship between luminance values in an image and a statistical value of the luminance values; an acquisition step of acquiring an image to be processed; a specification step of specifying an outlier pixel that deviates from a trend of output fluctuation indicated by the statistical value, derived on the basis of a luminance value of each pixel of the image and the relationship, using an evaluation value relating to fluctuation in a luminance value calculated for each pixel of the image; and a processing step of processing the image using a filter that reduces fluctuation in luminance values with respect to the outlier pixel in the image.

Alternatively, according to a second aspect of the embodiment, there is provided an image processing device including a processor, wherein the processor calculates a relationship between luminance values in an image and a statistical value of the luminance values, acquires an image to be processed, specifies an outlier pixel that deviates from a trend of output fluctuation indicated by the statistical value, derived on the basis of a luminance value of each pixel of the image and the relationship, using an evaluation value relating to fluctuation in a luminance value calculated for each pixel of the image, and processes the image using a filter that reduces fluctuation in luminance values with respect to the outlier pixel in the image.

Alternatively, according to a third aspect of the embodiment, there is provided an image processing program causing a processor to perform: a calculation step of calculating a relationship between luminance values in an image and a statistical value of the luminance values; an acquisition step of acquiring an image to be processed; a specification step of specifying an outlier pixel that deviates from a trend of output fluctuation indicated by the statistical value, derived on the basis of a luminance value of each pixel of the image and the relationship, using an evaluation value relating to fluctuation in a luminance value calculated for each pixel of the image; and a processing step of processing the image using a filter that reduces fluctuation in luminance values with respect to the outlier pixel in the image.

According to any of the first to third aspects, the evaluation value calculated for each pixel in the image is used to specify outlier pixels that deviate from the trend of an output fluctuation indicated by the statistical value calculated for the luminance value of each pixel and to perform a process of reducing fluctuations in luminance values for the specified outlier pixels. This allows noise reduction processing to be performed on pixels exhibiting statistically different behaviors of output fluctuations among pixels with various luminance values. As a result, stable noise reduction can be realized for images having various luminance values.

### Advantageous Effects of Invention

According to any aspect of the present embodiment, stable noise reduction can be realized in an image having various luminance values.

### Brief Description of Drawings

FIG. 1 is a configuration diagram of an X-ray imaging system 100 which is an image processing device according to a first embodiment.
FIG. 2 is a diagram illustrating a hardware configuration of a control device 121 in FIG. 1.
FIG. 3 is a block diagram illustrating a functional configuration of the control device 121 in FIG. 1.
FIG. 4 is a flowchart illustrating a procedure of noise reduction processing performed by the control device 121 in FIG. 1.
FIG. 5 is a diagram illustrating an example of X-ray images before and after noise reduction processing performed by the control device 121.
FIG. 6 is a block diagram illustrating a functional configuration of a control device 121A according to a second embodiment.
FIG. 7 is a graph illustrating a function of calculating a z-averaged log-likelihood MLH(1, 1) performed by a pixel specification unit 204A in FIG. 6.
FIG. 8 is a graph illustrating a function of calculating a z-averaged log-likelihood MLH(1, 1) performed by the pixel specification unit 204A in FIG. 6.
FIG. 9 is a graph illustrating a threshold setting process performed by the pixel specification unit 204A in FIG. 6.
FIG. 10 is a flowchart illustrating a procedure of noise reduction processing performed by the control device 121A in FIG. 6.

### Description of Embodiments

Hereinafter, embodiments of the present invention will be described in detail with reference to the accompanying drawings. Meanwhile, in each drawing, the same or equivalent parts are denoted by the same reference numerals, and thus duplicate description will be omitted.

### [First embodiment]

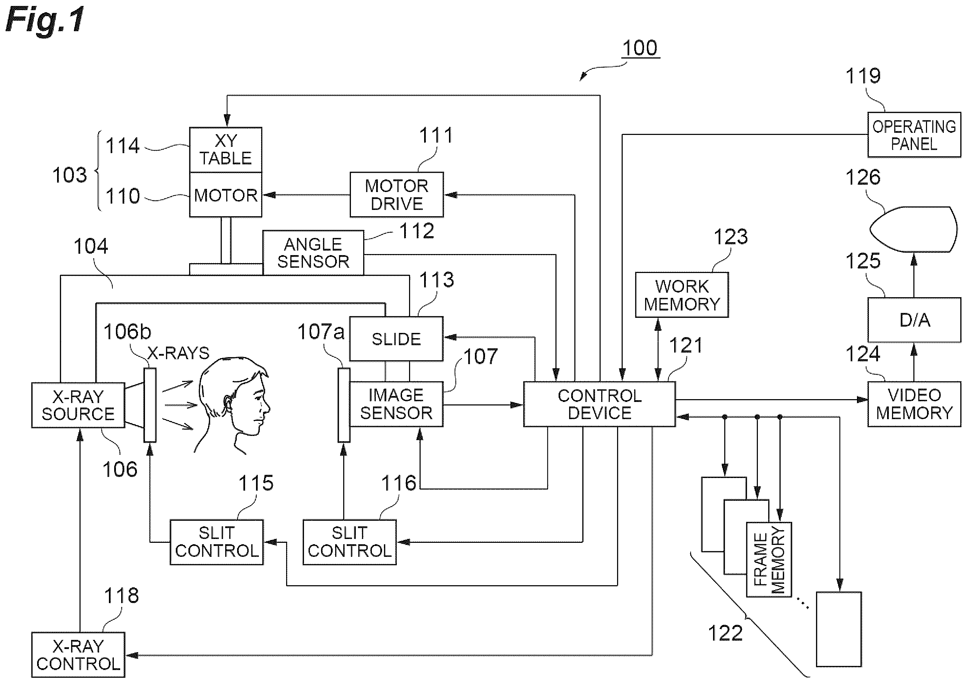

FIG. 1 is a configuration diagram of an X-ray imaging system 100 which is an image processing device according to a first embodiment. The X-ray imaging system 100 of the present embodiment includes a solid-state imaging device and an X-ray generation device, and X-rays output from the X-ray generation device and transmitted through the subject to be imaged are captured by the solid-state imaging device, which allows for inspection of the subject to be imaged.

In the X-ray imaging system 100 shown in the drawing, an X-ray generation device 106 generates X-rays toward the subject (subject to be imaged). The irradiation field of the X-rays generated from the X-ray generation device 106 is controlled by a primary slit plate 106b. The X-ray generation device 106 has an X-ray tube built-in, and the amount of X-ray radiated onto the subject is controlled by adjusting conditions such as the tube voltage, tube current, and power-on time of the X-ray tube. An X-ray imaging device (detector) 107 incorporates a CMOS solid-state imaging device having a plurality of pixels arranged two-dimensionally, and captures (detects) an X-ray image that has passed through the subject. A secondary slit plate 107a that restricts an X-ray incidence area is provided in front of the X-ray imaging device 107.

A rotating arm 104 holds the X-ray generation device 106 and the X-ray imaging device 107 so that these devices face each other, and rotates them around the subject during panoramic tomography. In addition, a slide mechanism 113 is provided to linearly displace the X-ray imaging device 107 relative to the subject during linear tomography. The rotating arm 104 is driven by an arm motor 110 constituting a rotary table, and the angle of rotation is detected by an angle sensor 112. In addition, the arm motor 110 is mounted on a movable portion of an XY table 114, and the center of rotation can be adjusted arbitrarily within a horizontal plane.

An image signal which is output from the X-ray imaging device 107 is first taken by a control device 121 and then stored in a frame memory 122. From image data stored in the frame memory 122, a tomographic image along an arbitrary tomographic plane is reconstructed through a predetermined calculation process. The reconstructed tomographic image is output to a video memory 124, converted into an analog signal by a DA converter 125, and then displayed on an image display unit 126 such as a cathode ray tube (CRT), serving various diagnostic purposes.

A work memory 123 required for signal processing is connected to the control device 121, and an operating panel 119 equipped with a panel switch, an X-ray irradiation switch, and the like is further connected thereto. In addition, the control device 121 is connected to a motor drive circuit 111 that drives the arm motor 110, slit control circuits 115 and 116 that control the opening range of the primary slit plate 106b and the secondary slit plate 107a, and an X-ray control circuit 118 that controls the X-ray generation device 106, and further outputs a signal for driving the X-ray imaging device 107.

The X-ray control circuit 118 can perform feedback control on the amount of X-rays radiated onto the subject on the basis of a signal captured by the X-ray imaging device 107.

In the X-ray imaging system 100 configured as described above, the X-ray imaging device 107 to be used is, for example, a flat panel sensor provided with a solid-state imaging element that includes a plurality of pixels arranged two-dimensionally on a light-receiving surface and a scintillator that covers the light-receiving surface of the solid-state imaging element. In addition, in this X-ray imaging system 100, the control device 121 performs predetermined noise reduction processing on the image data before the tomographic image is reconstructed, and then outputs the tomographic image reconstructed using the image data to the video memory 124.

A solid-state imaging element included in a flat panel sensor or the like using low-temperature poly silicon (LTPS) is excellent in terms of on-resistance (mobility) and can realize a high-speed response time, but it is known that, among a plurality of pixels arranged two-dimensionally, there are pixels called blinker pixels having larger output fluctuations than normal pixels. The behavior of output fluctuations of blinker pixels can be divided into two types: one in which the signal output suddenly fluctuates greatly and then immediately returns to normal output, and another in which the signal output fluctuates greatly for a certain period of time (several to several tens of frames) and then returns to normal output. In addition, even in general CMOS flat panel sensors other than low-temperature poly silicon (LTPS), there is also noise called random telegraph noise (RTN), which fluctuates irregularly over time.

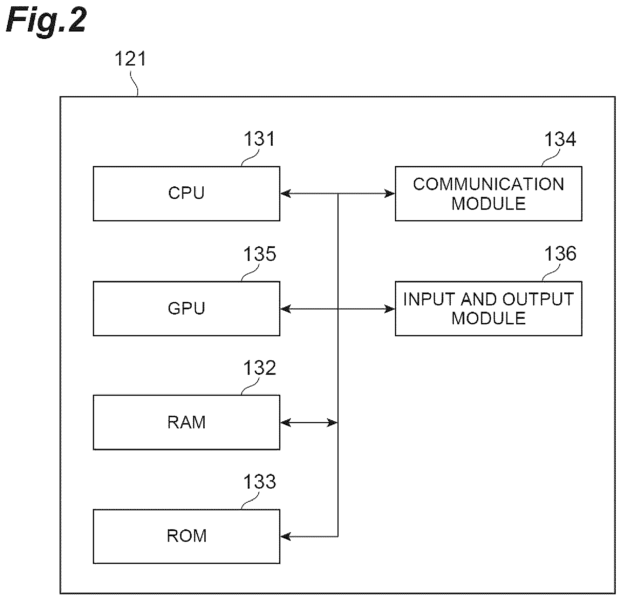

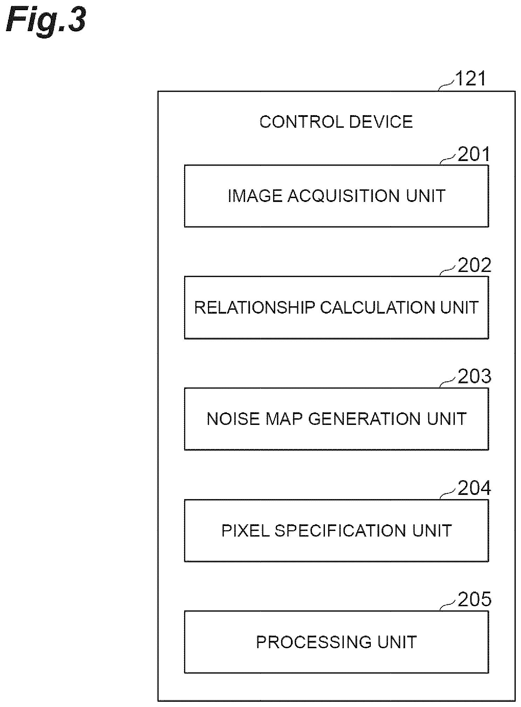

The function of the control device 121 regarding noise reduction processing (noise reduction function) will be described below. FIG. 2 is a block diagram illustrating a hardware configuration of the control device 121, and FIG. 3 is a block diagram illustrating a functional configuration for realizing the noise reduction function in the control device 121.

As shown in FIG. 2, the control device 121 is a computer or the like physically including a central processing unit (CPU) 131 and a graphic processing unit (GPU) 135 which are processors, a random access memory (RAM) 132 and a read only memory (ROM) 133 which are recording media, a communication module 134, an input and output module 136, and the like, which are electrically connected to each other. Meanwhile, the control device 121 may include a display, a keyboard, a mouse, a touch panel display, and the like as input and output devices, or may include a data recording device such as a hard disk drive or a semiconductor memory. In addition, the control device 121 may be constituted by a plurality of computers.

As shown in FIG. 3, the control device 121 includes an image acquisition unit 201, a relationship calculation unit 202, a noise map generation unit 203, a pixel specification unit 204, and a processing unit 205 as functional components related to the noise reduction function. Each functional unit of the control device 121 shown in FIG. 3 is realized by loading a program (an image processing program of the present embodiment) on the hardware such as the CPU 131, the GPU 135, and the RAM 132 to thereby bring the communication module 134, the input and output module 136, and the like into operation under the control of the CPU 131 and GPU 135 and read out and write data in the RAM 132. The CPU 131 and the GPU 135 of the control device 121 cause the control device 121 to function as each functional unit in FIG. 3 by executing this computer program, and sequentially execute processing corresponding to an image processing method to be described later. Meanwhile, the CPU 131 and the GPU 135 may be a single piece of hardware, or only one may be used. In addition, the CPU 131 and the GPU 135 may be implemented in a programmable logic such as an FPGA like a soft processor. The RAM or the ROM may also be a single piece of hardware, or may be built into a programmable logic such as an FPGA. Various types of data required for executing this computer program and various types of data generated by executing this computer program are all stored in a built-in memory such as the ROM 133 or the RAM 132, or a storage medium such as a hard disk drive or a solid-state drive.

The details of the function of each functional unit of the control device 121 will be described below.

The image acquisition unit 201 acquires image data of a two-dimensional X-ray image of the subject from the frame memory 122 (FIG. 1). The image acquisition unit 201 then performs a filtering process of reducing fluctuations in the luminance values (hereinafter also referred to pixel values) of all pixels included in the acquired X-ray image to thereby generate a noise-reduced image. The filtering process executed by the image acquisition unit 201 which will be used includes processing using a median filter, a bilateral filter, a Gaussian filter, or a non local means (NLM) filter, wavelet threshold processing, or noise reduction processing using AI.

The relationship calculation unit 202 calculates a relationship between luminance values in the noise-reduced image acquired by the image acquisition unit 201 and a statistical value of the luminance values as follows. In the present embodiment, a noise evaluation value obtained by evaluating the spread of noise values, that is, a noise sigma value which is the standard deviation of pixel values is used as the statistical value of pixel values. In addition, the variance of pixel values may be used as the statistical value of pixel values.

That is, the relationship calculation unit 202 calculates first relationship data indicating a relationship between the pixel value and the standard deviation of the pixel values through a simulation. The relational expression between the pixel value and the standard deviation of the pixel values used in the simulation by the relationship calculation unit 202 is expressed by the following Formulas (1) to (4). $\begin{matrix}Noise \\ = \sqrt{\begin{matrix}{\left({FE}_{m}MCQ\sqrt{\frac{cf}{{E}_{m} MCQ + {E}_{m} {M}_{Si} {rate}_{si}} ⋅ \left(Signal∗{C}_{s}-offset\right)}\right)}^{2} \\ + {\left({E}_{m}{M}_{si}\sqrt{{rate}_{si} \frac{cf}{{E}_{m} MCQ + {E}_{m} {M}_{Si} {rate}_{si}} ⋅ \left(Signal∗{C}_{s}-offset\right)}\right)}^{2}\end{matrix}} / \sqrt{N} / cf / {coeff}_{noise} \\ F = \sqrt{{{F}_{p}}^{2} + \frac{1}{MCQ}} \\ M = {Mcoeff}_{M} \\ {F}_{p} = {F}_{p} {coeff}_{Fp}\end{matrix}$

In Formulas (1) and (3), the variable Noise is information indicating the standard deviation of the pixel values, the variable Signal is information indicating the signal value (pixel value) of a pixel, the constant F is information indicating the noise factor, the variable Eₘ is information indicating the average energy of the X-rays, the constant M is information indicating the scintillator gain, the constant coeff_{M} is information indicating a multiplication factor adjustment coefficient for adjusting the scintillator gain, the constant C is information indicating the coupling efficiency between the solid-state imaging element and the scintillator in the X-ray imaging device 107, and the constant Q is information indicating the quantum efficiency of the solid-state imaging element.

In Formula (1), the constant cf is information indicating a conversion coefficient for converting the signal value of a pixel into an electric charge in the solid-state imaging element, and the constant R is information indicating readout noise in the solid-state imaging element. The conversion coefficient cf and the readout noise R are determined by the gain settings in the solid-state imaging element.

In Formula (1), the constant M_{Si} is information indicating the multiplication factor of the solid-state imaging element (silicon) when X-rays incident on the scintillator are incident on the solid-state imaging element without being converted into visible light, the constant rateₛᵢ is information indicating the silicon direct occurrence rate indicating the probability that X-rays incident on the scintillator are incident on the solid-state imaging element without being converted into visible light, the constant C_{S} is information indicating a shading correction value, and the constant offset is information indicating a camera offset indicating the offset value of the solid-state imaging element. In Formula (1), the constant N is information indicating the number of sensors. The constant N may be, for example, information indicating the number of solid-state imaging elements (the number of lines), or may be information indicating the binning setting in the solid-state imaging element. In Formula (1), coeffₙₒᵢₛₑ is a noise adjustment coefficient for adjusting noise. In Formulas (2) and (4), the constant Fₚ is information indicating a blur coefficient indicating blur, and the constant coeff_{Fp} is information indicating a blur coefficient adjustment coefficient for adjusting the coefficient indicating blur.

Here, when Formulas (1) to (4) are used, the relationship calculation unit 202 substitutes the pixel value of each pixel in the noise-reduced image generated by the image acquisition unit 201 for the variable Signal, and sets other parameters in Formulas (1) to (4) on the basis of information input by a user through the operating panel 119. The relationship calculation unit 202 then acquires the variable Noise calculated using Formulas (1) to (4) as the numerical value of the standard deviation of the pixel values of each pixel in the noise-reduced image. Meanwhile, the parameters in Formulas (1) to (4) may be set on the basis of information accepted from a user, or may be set by reading out data stored in the RAM 132, the ROM 133, or the like within the control device 121 on the basis of the accepted information.

For example, the relationship calculation unit 202 accepts input of the following information from the user.
· Measurement camera model
· Gain setting
· Number of binnings, number of lines
· X-ray average energy Eₘ
· Camera offset offset
· Silicon direct occurrence rate rateₛᵢ
· Shading correction value C_{S}
· Noise adjustment coefficient coeffₙₒᵢₛₑ
· Multiplication factor adjustment coefficient coeff_{M}
· Blur coefficient adjustment coefficient coeff_{Fp}

The relationship calculation unit 202 reads out parameters such as the scintillator gain M, the coupling efficiency C, the blur coefficient Fₚ, and the quantum efficiency Q which are stored in association with the "measurement camera model" from within the control device 121 on the basis of the accepted information "measurement camera model." For example, in a case where the "measurement camera model" is "camera 1," the relationship calculation unit 202 reads out the parameters of the scintillator gain M = 10, the coupling efficiency C = 0.7, the blur coefficient Fₚ = 0.9, and the quantum efficiency Q = 0.9 which are associated with "camera 1," and in a case where the "measurement camera model" is "camera 2," the relationship calculation unit reads out the parameters of the scintillator gain M = 100, the coupling efficiency C = 0.8, the blur coefficient Fₚ = 0.8, and the quantum efficiency Q = 0.7 which are associated with "camera 2." In the same way, the relationship calculation unit 202 reads out parameters such as the conversion coefficient cf and the readout noise R which are stored in association with the "gain setting" from within the control device 121 on the basis of the accepted information "gain setting," and reads out parameters such as the number of sensors N which are stored in association with "number of binnings, number of lines" from within the control device 121 on the basis of the accepted information "number of binnings, number of lines." Further, the relationship calculation unit 202 sets other parameters such as the X-ray average energy Eₘ on the basis of information accepted from a user.

In addition, the relationship calculation unit 202 may derive the first relationship data indicating the relationship between pixel values and the standard deviation of the pixel values on the basis of an image obtained by actual image capturing. As an example, the relationship calculation unit 202 may acquire an X-ray image (test image) captured by irradiating a jig with X-rays. As the jig, a flat plate-like member or the like of which the thickness and material are known is used. The relationship calculation unit 202 may derive relationship data indicating the relationship between the pixel values of the noise-reduced image generated from the acquired test image and the standard deviation of the pixel values.

The derivation of the first relationship data indicating the relationship between pixel values and the standard deviation of the pixel values from the X-ray image obtained by capturing an image of the jig which is performed by the relationship calculation unit 202 will be described. For the jig, for example, a member of which the thickness changes stepwise in one direction can be used. First, in the noise-reduced image obtained from the test image obtained by capturing an image of the jig, the relationship calculation unit 202 derives a pixel value in a case where there is no noise for each step of the jig (hereinafter referred to as a true pixel value), and derives the standard deviation of the pixel values on the basis of the true pixel value. Specifically, the relationship calculation unit 202 derives the average value of the pixel values at a certain step of the jig. The relationship calculation unit 202 then uses the derived average value of the pixel values as the true pixel value at that step. In that step, the relationship calculation unit 202 derives the difference between each pixel value and the true pixel value as a noise value. The relationship calculation unit 202 derives the standard deviation of the pixel values from the derived noise value for each pixel value.

The relationship calculation unit 202 then derives the first relationship data on the basis of the relationship between true pixel values and the standard deviation of the pixel values. Specifically, the relationship calculation unit 202 derives the true pixel value and the standard deviation of the pixel values for each step of the jig. The relationship calculation unit 202 plots the relationship between the derived true pixel values and the standard deviation of the pixel values on a graph and draws an approximation curve to derive a relationship graph indicating the relationship between pixel values and the standard deviation of the pixel values. Meanwhile, for the approximation curve, exponential approximation, linear approximation, logarithmic approximation, polynomial approximation, power approximation, or the like is used.

The relationship calculation unit 202 may use a plurality of X-ray images captured with varying luminance in a state where there is no subject, instead of the X-ray image obtained by capturing an image of a jig as the test image. In this case, a plurality of test images obtained while changing the output intensity of the X-ray generation device 106 or a plurality of test images obtained while changing the exposure time of the X-ray imaging device 107 are used. The relationship calculation unit 202 can derive a relationship graph indicating the relationship between the pixel value and the standard deviation of the pixel values in the same way as the procedure described above.

The noise map generation unit 203 specifies the value of the standard deviation from the pixel value for each pixel in the noise-reduced image on the basis of the first relationship data indicating the relationship between the pixel value of each pixel in the noise-reduced image and the standard deviation of the pixel values calculated by the relationship calculation unit 202. The noise map generation unit 203 generates a noise map which is data in which the specified standard deviation is associated with each pixel in the noise-reduced image.

Based on the noise map generated by the noise map generation unit 203, the pixel specification unit 204 specifies, from among all pixels of the X-ray image, outlier pixels that deviate from the trend of an output fluctuation indicated by the statistical value of pixel values specified by the first relationship data. Based on the noise map, these outlier pixels are pixels that may have outlier values deviating from the trend of an output fluctuation indicated by the statistical value of pixel values specified by the first relationship data, from among all pixels of the X-ray image. These outlier pixels may include the blinker pixels described above.

That is, the pixel specification unit 204 calculates an evaluation value Th(i, j) relating to the fluctuation in the pixel value f for each pixel in the X-ray image acquired by the image acquisition unit 201 using the following Formula (5).
[Math. 2] $Th \left(i, j\right) = {\sum }_{n = - w}^{w} {\sum }_{m = - w}^{w} f \left(i, j\right) - f \left(i+m,j+n\right)$

Here, in the above (5), i is an integer representing the horizontal position of the pixel to be calculated, j is an integer representing the vertical position of the pixel to be calculated, m and n are variables for defining the range of surrounding pixels, and w is a constant (integer) for defining the range of surrounding pixels. The pixel specification unit 204 calculates the cumulative value of differences between the pixel value f(i, j) of a specific pixel and the pixel values f(i+m, j+n) of pixels surrounding the specific pixel as the evaluation value Th(i, j) of the specific pixel. By using such a calculation formula, it is possible to calculate an evaluation value that evaluates how much the pixel value f of each pixel fluctuates compared to the surrounding pixels.

The pixel specification unit 204 then determines whether each pixel corresponds to an outlier pixel as follows. The pixel specification unit 204 compares the evaluation value Th(i, j) of the pixel to be determined with a threshold K×σ(i, j) determined from the standard deviation σ(i, j) of each pixel read out from the noise map, and determines that the pixel to be determined corresponds to an outlier pixel in a case where the evaluation value Th(i, j) is greater than the threshold (that is, in a case where the evaluation value Th is an outlier value). In this case, the coefficient K for determining the threshold is set to a value in the range of K = 8×3 to 8×5 when the constant w = 1. Such a determination is repeated for all pixels of the X-ray image to specify outlier pixels from all pixels.

The processing unit 205 targets an X-ray image in which outlier pixels have been specified and replaces the pixel values of the outlier pixels in the original X-ray image acquired by the image acquisition unit 201 with the pixel values of the corresponding pixels in the noise-reduced image. In this case, the processing unit 205 repeats the pixel value replacement for all outlier pixels specified in the X-ray image. Such a process is a process equivalent to executing a filtering process on a plurality of outlier pixels in the X-ray image. In addition, the processing unit 205 sequentially stores the X-ray image on which the filtering process has been executed in the frame memory 122 (FIG. 1). The X-ray image stored in the frame memory 122 is processed by the control device 121 to be reconstructed into a tomographic image.

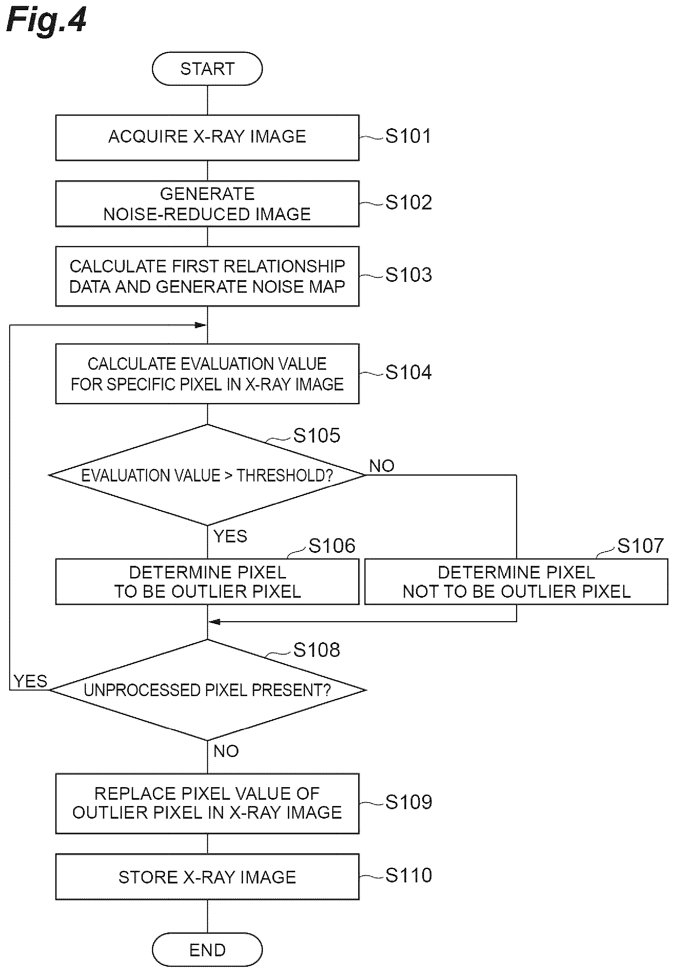

Next, a description will be given of a procedure for noise reduction processing on an X-ray image using the control device 121, that is, a flow of the image processing method according to the present embodiment. FIG. 4 is a flowchart illustrating a procedure for noise reduction processing performed by the control device 121.

First, in the control device 121, the image acquisition unit 201 acquires an X-ray image from the frame memory 122 (step S101, acquisition step). Next, the image acquisition unit 201 performs the filtering process on the X-ray image to generate a noise-reduced image (step S102, processing step). Next, the relationship calculation unit 202 generates the first relationship data indicating the relationship between the pixel values and the standard deviation of the pixel values, and generates a noise map, that is data in which the standard deviation is associated with each pixel in the noise-reduced image, on the basis of the first relationship data (step S103: calculation step).

Next, the pixel specification unit 204 of the control device 121 calculates the evaluation value Th(i, j) for the specific pixel in the X-ray image acquired in step S101 (step S104). The pixel specification unit 204 then compares the evaluation value Th(i, j) of the specific pixel with a threshold determined from the standard deviation σ(i, j) associated with the specific pixel read out from the noise map (step S105). As a result of the comparison, in a case where it is determined that the evaluation value Th(i, j) is greater than the threshold (step S105; Yes), the specific pixel is determined to be an outlier pixel. On the other hand, in a case where it is determined that the evaluation value Th(i, j) is equal to or less than the threshold (step S105; No), the specific pixel is determined not to be an outlier pixel. The processes from step S104 to S107 are repeated for all pixels in the X-ray image (step S108, specification step).

Further, the processing unit 205 of the control device 121 replaces the pixel values of the outlier pixels in the X-ray image with the pixel values of the corresponding pixels in the noise-reduced image (step S109, processing step). Finally, the processing unit 205 stores the image data of the X-ray image on which the filtering process has been executed in the frame memory 122 (step S110).

In the control device 121 according to the first embodiment described above, the evaluation value calculated for each pixel in the X-ray image is used to specify outlier pixels that deviate from the trend of an output fluctuation indicated by the statistical value calculated for the luminance value of each pixel and to perform a process of reducing fluctuations in luminance values for the specified outlier pixels. This allows noise reduction processing to be performed on pixels exhibiting statistically different behaviors of output fluctuations among pixels with various luminance values. As a result, stable noise reduction can be realized for images having various luminance values. In Particular, the blinker pixels included in the solid-state imaging element of the X-ray imaging device 107 can be stably specified, and the filtering process can be performed only on pixels corresponding to the blinker pixels, which makes it possible to prevent degradation of resolution in the X-ray image.

In the first embodiment, the outlier pixels are specified in accordance with a result of comparing the evaluation value calculated on the basis of the luminance value of each pixel in the X-ray image with the statistical value of the luminance values. Specifically, in the first embodiment, whether a specific pixel is an outlier pixel is determined in accordance with the result of comparing the cumulative value of differences between the luminance value of the specific pixel in the X-ray image and the luminance values of the pixels surrounding the specific pixel with the statistical value of the luminance value of the specific pixel. This allows pixels exhibiting statistically different behaviors of output fluctuations among pixels with various luminance values to be specified through simple calculation.

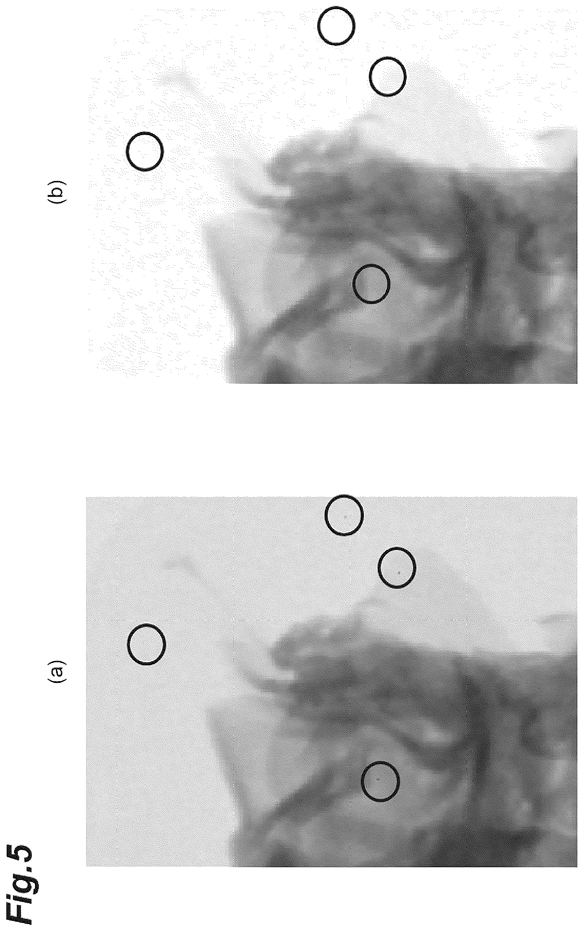

FIG. 5 shows an example of an X-ray image processed by the control device 121 according to the first embodiment, in which part (a) shows the X-ray image before noise reduction processing, and part (b) shows the X-ray image after noise reduction processing has been performed on the X-ray image shown in part (a). In these X-ray images, locations specified as outlier pixels are marked with circles. In this processing example, it has been confirmed that noise is reduced only in pixel portions having noise with large fluctuations in luminance value compared to normal noise, and it has also been understood that a decrease in resolution is reduced.

### [Second embodiment]

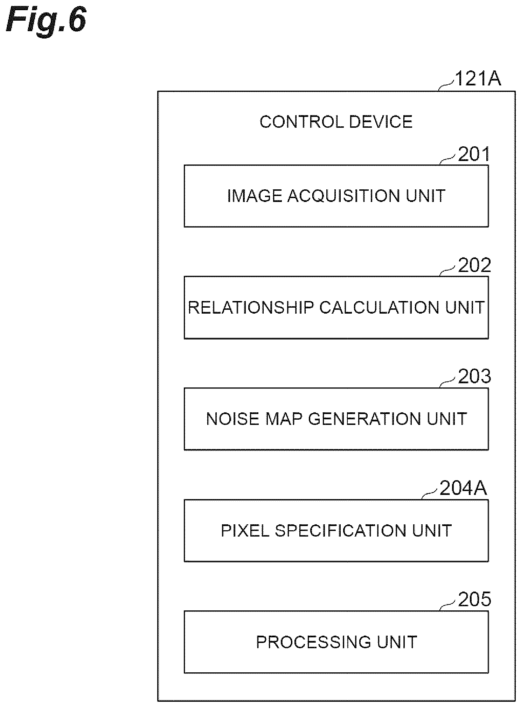

FIG. 6 is a block diagram illustrating a functional configuration of a control device 121A which is an image processing device according to a second embodiment. The control device 121A differs from the control device 121 according to the first embodiment in the function of specifying outlier pixels performed by the pixel specification unit 204A. Hereinafter, a description will be given with focus on differences from the first embodiment.

The pixel specification unit 204A processes a plurality of X-ray images captured consecutively over time and acquired by the image acquisition unit 201, and a plurality of noise maps generated by the noise map generation unit 203 for the plurality of X-ray images.

Specifically, the pixel specification unit 204A generates a plurality of noise-extracted images obtained by extracting noise on the basis of a plurality of X-ray images as follows. The pixel specification unit 204A generates a noise-extracted image by subtracting the pixel value of each pixel in the noise-reduced image generated on the basis of the X-ray image from the pixel value of each pixel in the X-ray image.

Further, the pixel specification unit 204A calculates an evaluation value relating to the probability of noise occurrence for each pixel in the plurality of noise-extracted images as follows. First, the pixel specification unit 204A reads out the standard deviation σ(i, j, z) associated with a specific pixel on the basis of the pixel value Noise(i, j, z) (i is an integer representing the horizontal position of the pixel, j is an integer representing the vertical position of the pixel, and z is an integer representing the order of the image in the time direction) of the specific pixel in the noise-extracted image and the noise map generated from the X-ray image which is the source of the noise-extracted image. The pixel specification unit 204A calculates the noise occurrence probability p(i, j, z) of the specific pixel determined from the normal distribution using the following Formula (6).
[Math. 3] $p \left(i, j, z\right) = \frac{1}{σ \left(i, j, z\right) \sqrt{2 π}} exp \left(-\frac{{\left(Noise\left(i, j, z\right)\right)}^{2}}{2 σ {\left(i, j, z\right)}^{2}}\right)$

Similarly, the pixel specification unit 204A continuously calculates a plurality of noise occurrence probabilities p(i, j, z) (z = 1, 2, ..., K) of a specific pixel in K noise-extracted images on the basis of the pixel value Noise(i, j, z) (z = 1, 2, ..., K) of the specific pixel and K noise maps.

Next, the pixel specification unit 204A calculates the log-likelihood LH(i, j, z) of each noise occurrence probability from the plurality of noise occurrence probabilities p(i, j, z) (z = 1, 2, ..., K) of the specific pixel using the following Formula (7) $LH \left(i, j, z\right) = - log \left\{p\left(i, j, z\right)\right\}$ and then calculates the z-averaged log-likelihood MLH(i, j) obtained by averaging the log-likelihood in the time direction (z direction) using the following Formula (8).
[Math. 4] $MLH \left(i, j\right) = \frac{1}{K} {\sum }_{z = 1}^{K} LH \left(i, j, z\right)$

This z-averaged log-likelihood MLH(i, j) is an evaluation value relating to the probability of noise occurrence for a specific pixel. Further, the pixel specification unit 204A compares the z-averaged log-likelihood MLH(i, j) calculated for the specific pixel with a threshold set in advance, and determines that the specific pixel corresponds to an outlier pixel in a case where the z-averaged log-likelihood MLH(i, j) is greater than the threshold. The pixel specification unit 204 repeats the above-described determination process for each pixel in the plurality of noise-extracted images.

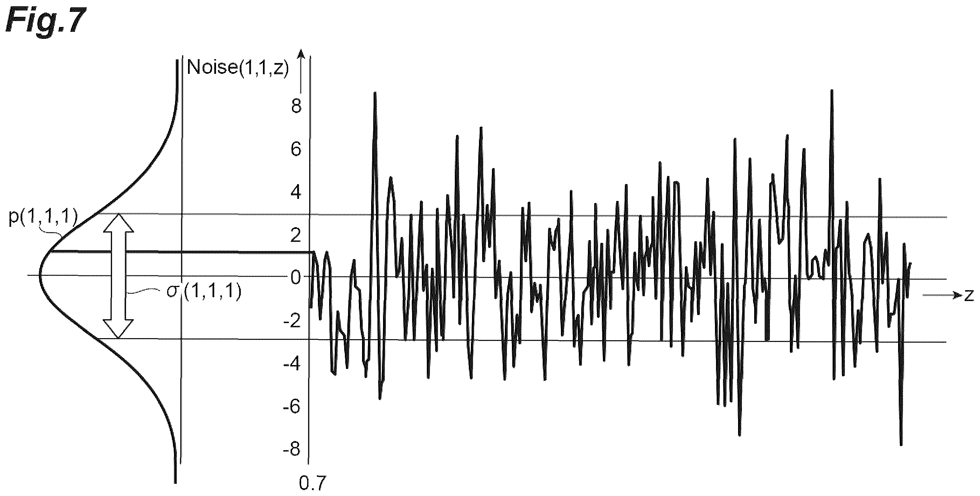

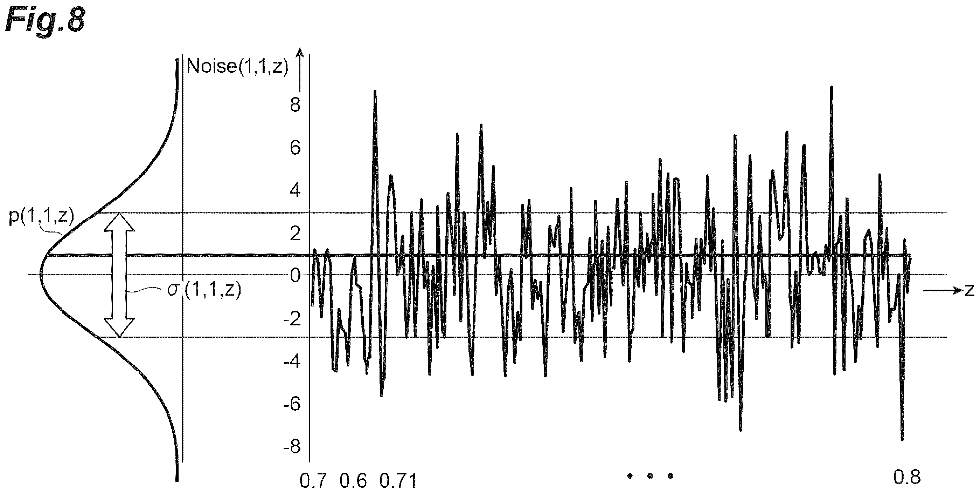

FIGS. 7 and 8 are graphs illustrating a function of calculating the z-averaged log-likelihood MLH(1, 1) performed by the pixel specification unit 204A. In this way, the noise occurrence probabilities 0.7, 0.6, 0.71, ..., 0.8 are calculated for each pixel value Noise(1, 1, z) (z = 1, 2, ..., K) of a specific pixel in the plurality of noise-extracted images. In this case, when each noise occurrence probability p(1, 1, z) (z = 1, 2, ..., K) is calculated, a normal distribution probability formula p(1, 1, z) is used, which is set on the basis of the standard deviation σ(1, 1, z) determined from the noise map corresponding to the order of each noise-extracted image. Further, based on the noise occurrence probabilities calculated in a time series in this way, the z-averaged log-likelihood MLH(1, 1), which is an evaluation value relating to the noise occurrence probability of a specific pixel, is calculated.

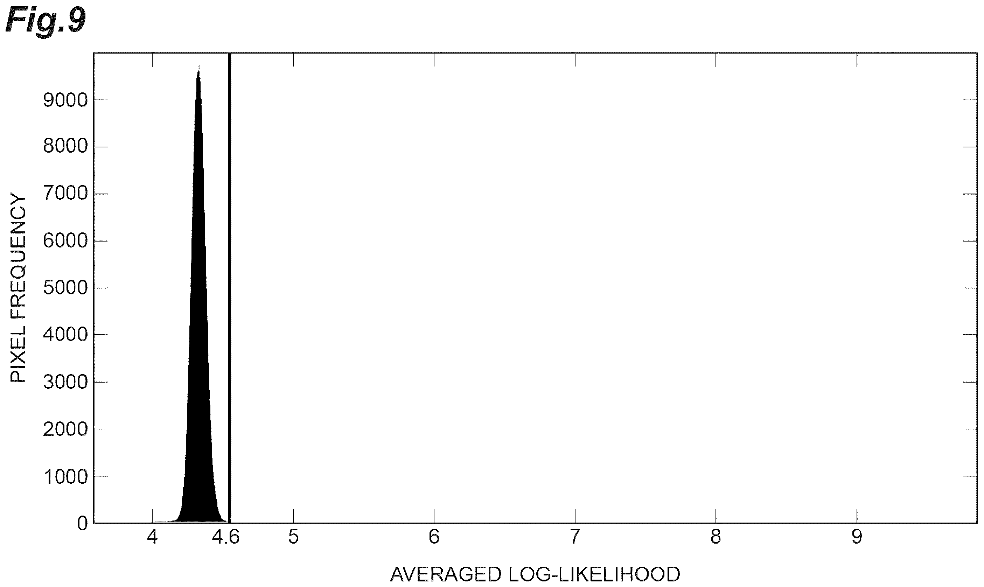

Meanwhile, the threshold referred to when the pixel specification unit 204A specifies an outlier pixel may be set in advance, or may be determined each time. FIG. 9 is a graph illustrating a threshold setting process performed by the pixel specification unit 204A. The pixel specification unit 204A calculates the z-averaged log-likelihood MLH for a plurality of noise-extracted images generated from a plurality of X-ray images acquired in advance, and aggregates the pixel frequency for each value of the z-averaged log-likelihood MLH. The pixel specification unit 204A then sets the log-likelihood at which the pixel frequency value falls below an arbitrary value as the threshold. In the example shown in FIG. 9, the threshold is set to "4.6" which is a value greater than the peak position of the histogram.

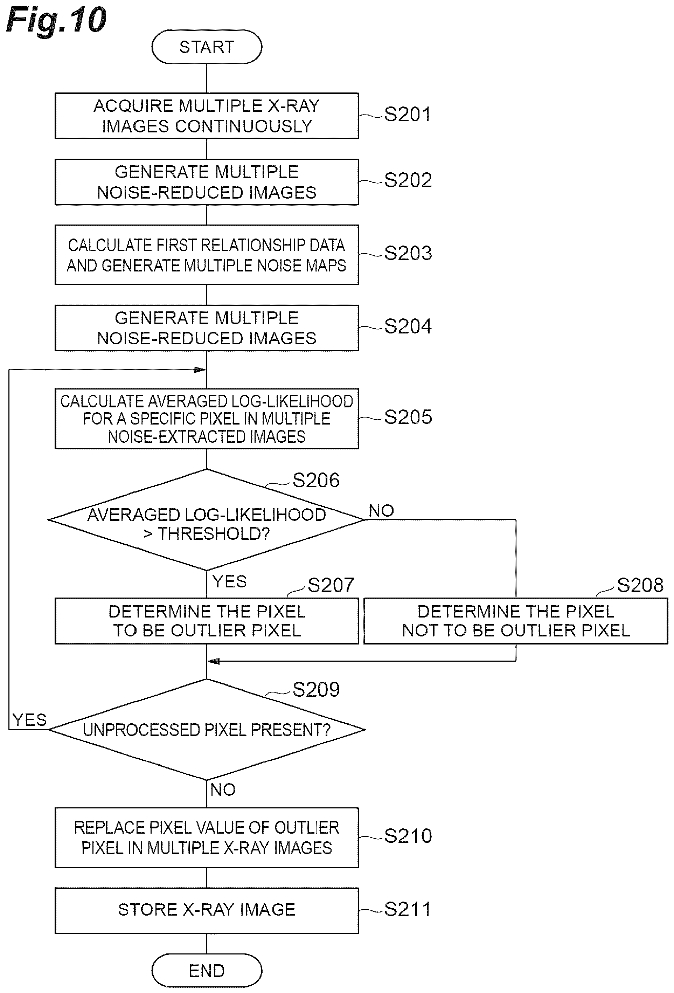

Next, a procedure for noise reduction processing on X-ray images using the control device 121A, that is, a flow of an image processing method according to the second embodiment, will be described. FIG. 10 is a flowchart illustrating a procedure for noise reduction processing performed by the control device 121A.

First, in the control device 121A, the image acquisition unit 201 acquires a plurality of X-ray images that have been continuously captured from the frame memory 122 (step S201, acquisition step). Next, the image acquisition unit 201 performs the filtering process on the plurality of X-ray images to generate a plurality of noise-reduced images (step S202, processing step). As the filtering process, for example, a process using a 3×3 median filter is performed. Next, the relationship calculation unit 202 generates the first relationship data indicating the relationship between pixel values and the standard deviation of the pixel values, and generates a plurality of noise maps which are data in which the standard deviation is associated with each pixel in the plurality of noise-reduced images on the basis of the first relationship data (step S203: calculation step).

Next, the pixel specification unit 204A of the control device 121A generates a plurality of noise-extracted images on the basis of the plurality of X-ray images and the plurality of noise-reduced images (step S204). Next, the pixel specification unit 204A calculates the z-averaged log-likelihood MLH for specific pixels in the plurality of noise-extracted images using the plurality of noise maps (step S205). The pixel specification unit 204A then compares the z-averaged log-likelihood MLH(i, j) of the specific pixel with a threshold (step S206). As a result of the comparison, in a case where it is determined that the z-averaged log-likelihood MLH(i, j) is greater than the threshold (step S206; Yes), the specific pixel is determined to be an outlier pixel. On the other hand, in a case where it is determined that the z-averaged log-likelihood MLH(i, j) is equal to or less than the threshold (step S206; No), the specific pixel is determined not to be an outlier pixel. The processes from step S205 to S208 are repeated for all pixels in the X-ray image (step S209, specification step).

Further, the processing unit 205 of the control device 121A replaces the pixel values of the outlier pixels in the plurality of X-ray images with the pixel values of the corresponding pixels in the noise-reduced images corresponding to the plurality of X-ray images (step S210, processing step). Finally, the processing unit 205 stores the image data of the plurality of X-ray images on which the filtering process has been executed in the frame memory 122 (step S211).

In the control device 121A according to the second embodiment described above, an evaluation value relating to the probability is calculated from the luminance value of each pixel in the X-ray image and the statistical value of the luminance values. According to such a configuration, pixels exhibiting statistically different behaviors of output fluctuations among pixels with various luminance values can be specified with a high degree of accuracy.

In particular, in the control device 121A, a plurality of X-ray images captured consecutively over time are acquired, a plurality of evaluation values are calculated for specific pixels in the plurality of X-ray images, and whether the specific pixel is an outlier pixel is determined on the basis of the plurality of evaluation values. According to such a configuration, pixels exhibiting statistically different behaviors of output fluctuations among pixels with various luminance values can be stably specified without omission. That is, by evaluating the behavior of pixel luminance fluctuations in the time direction as well, blinker pixels which are buried in noise can also be stably specified. When a plurality of X-ray images actually captured were processed using the control device 121A, it was confirmed that all five blinker pixels that were causing artifacts in tomographic images based on the X-ray images could be specified.

### [Modification example]

Although various embodiments of the present invention have been described above, the embodiments of the present invention are not limited to the above-described embodiments.

In the control device 121 according to the first embodiment and the control device 121A according to the second embodiment described above, outlier pixels including blinker pixels in the X-ray image are specified, but objects to be processed and specified are not limited thereto. For example, in an image captured by an image sensor such as a CCD or CMOS, pixels that generate other types of noise such as burst noise may be specified.

In addition, the noise map generation unit 203 of the control devices 121 and 121A generates a noise map by specifying the value of standard deviation from the pixel value of each pixel in the noise-reduced image, but it may generate a noise map by specifying the value of standard deviation from the pixel values of the original X-ray image.

The pixel specification unit 204A of the control device 121A according to the second embodiment calculates the log-likelihood as the evaluation value, but the likelihood may be used instead of the log-likelihood. In addition, the pixel specification unit 204A may derive the value of the noise occurrence probability used when the likelihood is calculated using the formula of Gaussian distribution, Poisson distribution, or t-distribution, or actually measured probability distribution. In addition, the pixel specification unit 204A calculates the average value of the log-likelihood in the z direction as the evaluation value, but may calculate the median, maximum value, minimum value, or the like instead of the average value.

In addition, the pixel specification unit 204A of the control device 121A according to the second embodiment calculates an evaluation value for a specific pixel in a plurality of X-ray images acquired continuously, but it may calculate a plurality of evaluation values for a plurality of pixels included in a pixel group in the same X-ray image, and use the average value of the plurality of evaluation values, or the like, to determine whether the pixel group includes outlier pixels. For example, the pixel specification unit 204 may perform the determination on a pixel group including pixels in the same column of the X-ray image, may perform the determination on a pixel group including pixels in the same row of the X-ray image, or may perform the determination on a pixel group in any range in which an abnormality is to be determined.

In addition, upon the determination of an outlier pixel, the pixel specification unit 204A may determine whether the evaluation value is greater than a threshold, may determine whether the determination value is less than the threshold, or may determine whether the determination value is within the range of two thresholds. In addition, the threshold for determining an outlier pixel may be changed for each pixel position taking into consideration that there are pixel positions where blinker pixels are likely to appear.

In the first aspect, the specification step may include specifying the outlier pixel in accordance with a result of comparing the evaluation value calculated on the basis of the luminance value of each pixel of the image with the statistical value of the luminance value. In the second aspect, the processor may specify the outlier pixel in accordance with a result of comparing the evaluation value calculated on the basis of the luminance value of each pixel of the image with the statistical value of the luminance value. This allows pixels exhibiting statistically different behaviors of output fluctuations among pixels with various luminance values to be specified through simple calculation.

In addition, in the first aspect, the specification step may include determining whether a specific pixel in the image is an outlier pixel in accordance with a result of comparing a cumulative value of differences between a luminance value of the specific pixel and luminance values of pixels surrounding the specific pixel with a statistical value of the luminance value of the specific pixel. In addition, in the second aspect, the processor may determine whether a specific pixel in the image is an outlier pixel in accordance with a result of comparing a cumulative value of differences between a luminance value of the specific pixel and luminance values of pixels surrounding the specific pixel with a statistical value of the luminance value of the specific pixel. According to the above configuration, pixels exhibiting statistically different behaviors of output fluctuations among pixels with various luminance values can be specified through simple calculation.

In the first aspect, the specification step may include calculating the evaluation value relating to probability from the luminance value of each pixel of the image and the statistical value of the luminance value. In addition, in the second aspect, the processor may calculate the evaluation value relating to probability from the luminance value of each pixel of the image and the statistical value of the luminance value. According to the above configuration, pixels exhibiting statistically different behaviors of output fluctuations among pixels with various luminance values can be specified with a high degree of accuracy.

Further, in the first aspect, the acquisition step may include acquiring a plurality of the images consecutively over time, and the specification step may include calculating a plurality of the evaluation values for specific pixels in the plurality of images, and determining whether the specific pixels are outlier pixels on the basis of the plurality of evaluation values. Further, in the second aspect, the processor may acquire a plurality of the images consecutively over time, calculate a plurality of the evaluation values for specific pixels in the plurality of images, and determine whether the specific pixels are outlier pixels on the basis of the plurality of evaluation values. According to such a configuration, pixels exhibiting statistically different behaviors of output fluctuations among pixels with various luminance values can be stably specified without omission.

In the first aspect, the specification step may include calculating a plurality of the evaluation values for a plurality of pixels included in a pixel group in the image, and determining whether the pixel group includes outlier pixels on the basis of the plurality of evaluation values. In addition, in the second aspect, the processor may calculate a plurality of the evaluation values for a plurality of pixels included in a pixel group in the image, and determine whether the pixel group includes outlier pixels on the basis of the plurality of evaluation values. In this case, pixel groups with statistically different output fluctuation behaviors are specified. As a result, stable noise reduction can be realized for images in which output fluctuations are likely to occur in units of pixel groups.

In the first aspect or the second aspect, the statistical value may be a standard deviation or a variance. In addition, in the second aspect, a detector configured to detect the image with respect to a subject may be further included.

The image processing method of the embodiment is [1] "an image processing method comprising: a calculation step of calculating a relationship between luminance values in an image and a statistical value of the luminance values; an acquisition step of acquiring an image to be processed; a specification step of specifying an outlier pixel that deviates from a trend of output fluctuation indicated by the statistical value, derived on the basis of the luminance value of each pixel of the image and the relationship, using an evaluation value relating to fluctuation in a luminance value calculated for each pixel of the image; and a processing step of processing the image using a filter that reduces fluctuation in luminance values with respect to the outlier pixel in the image."

The image processing method of the embodiment may be [2] "the image processing method according to the above [1], wherein the specification step includes specifying the outlier pixel in accordance with a result of comparing the evaluation value calculated on the basis of the luminance value of each pixel of the image with the statistical value of the luminance value."

The image processing method of the embodiment may be [3] "the image processing method according to the above [2], wherein the specification step includes determining whether a specific pixel in the image is an outlier pixel in accordance with a result of comparing a cumulative value of differences between a luminance value of the specific pixel and luminance values of pixels surrounding the specific pixel with a statistical value of the luminance value of the specific pixel."

The image processing method of the embodiment may be [4] "the image processing method according to the above [1], wherein the specification step includes calculating the evaluation value relating to probability from the luminance value of each pixel of the image and the statistical value of the luminance value."

The image processing method of the embodiment may be [5] "the image processing method according to the above [4], wherein the acquisition step includes acquiring a plurality of the images consecutively over time, and the specification step includes calculating a plurality of the evaluation values for specific pixels in the plurality of images, and determining whether the specific pixels are outlier pixels on the basis of the plurality of evaluation values."

The image processing method of the embodiment may be [6] "the image processing method according to the above [4] or [5], wherein the specification step includes calculating a plurality of the evaluation values for a plurality of pixels included in a pixel group in the image, and determining whether the pixel group includes outlier pixels on the basis of the plurality of evaluation values."

The image processing method of the embodiment may be [7] "the image processing method according to any one of the above [1] to [6], wherein the statistical value is a standard deviation or a variance."

The image processing device of the embodiment may be [8] "an image processing device comprising a processor, wherein the processor calculates a relationship between luminance values in an image and a statistical value of the luminance values, acquires an image to be processed, specifies an outlier pixel that deviates from a trend of output fluctuation indicated by the statistical value, derived on the basis of the luminance value of each pixel of the image and the relationship, using an evaluation value relating to fluctuation in a luminance value calculated for each pixel of the image, and processes the image using a filter that reduces fluctuation in luminance values with respect to the outlier pixel in the image."

The image processing device of the embodiment may be [9] "the image processing device according to the above [8], wherein the processor specifies the outlier pixel in accordance with a result of comparing the evaluation value calculated on the basis of the luminance value of each pixel of the image with the statistical value of the luminance value."

The image processing device of the embodiment may be [10] "the image processing device according to the above [9], wherein the processor determines whether a specific pixel in the image is an outlier pixel in accordance with a result of comparing a cumulative value of differences between a luminance value of the specific pixel and luminance values of pixels surrounding the specific pixel with a statistical value of the luminance value of the specific pixel."

The image processing device of the embodiment may be [11] "the image processing device according to the above [8], wherein the processor calculates the evaluation value relating to probability from the luminance value of each pixel of the image and the statistical value of the luminance value."

The image processing device of the embodiment may be [12] "the image processing device according to the above [11], wherein the processor acquires a plurality of the images consecutively over time, calculates a plurality of the evaluation values for specific pixels in the plurality of images, and determines whether the specific pixels are outlier pixels on the basis of the plurality of evaluation values."

The image processing device of the embodiment may be [13] "the image processing device according to the above [11] or [12], wherein the processor calculates a plurality of the evaluation values for a plurality of pixels included in a pixel group in the image, and determines whether the pixel group includes outlier pixels on the basis of the plurality of evaluation values."

The image processing device of the embodiment may be [14] "the image processing device according to any one of the above [8] to [13], wherein the statistical value is a standard deviation or a variance."

The image processing device of the embodiment may be [15] "the image processing device according to any one of the above [8] to [14], further comprising a detector configured to detect the image with respect to a subject."

### Reference Signs List

100 X-ray imaging system, 131 CPU (processor), 135 GPU (processor), 107 X-ray imaging device (detector), 106 X-ray generation device, 121, 121A Control device, 201 Image acquisition unit, 204, 204A Pixel specification unit, 202 Relationship calculation unit, 203 Noise map generation unit, 205 Processing unit

## Claims

1. An image processing method comprising:
a calculation step of calculating a relationship between luminance values in an image and a statistical value of the luminance values;
an acquisition step of acquiring an image to be processed;
a specification step of specifying an outlier pixel that deviates from a trend of output fluctuation indicated by the statistical value, derived on the basis of a luminance value of each pixel of the image and the relationship, using an evaluation value relating to fluctuation in the luminance value calculated for each pixel of the image; and
a processing step of processing the image using a filter that reduces fluctuation in luminance values with respect to the outlier pixel in the image.

2. The image processing method according to claim 1, wherein the specification step includes specifying the outlier pixel in accordance with a result of comparing the evaluation value calculated on the basis of the luminance value of each pixel of the image with the statistical value of the luminance value.

3. The image processing method according to claim 2, wherein the specification step includes determining whether a specific pixel in the image is an outlier pixel in accordance with a result of comparing a cumulative value of differences between a luminance value of the specific pixel and luminance values of pixels surrounding the specific pixel with a statistical value of the luminance value of the specific pixel.

4. The image processing method according to claim 1, wherein the specification step includes calculating the evaluation value relating to probability from the luminance value of each pixel of the image and the statistical value of the luminance value.

5. The image processing method according to claim 4, wherein the acquisition step includes acquiring a plurality of the images consecutively over time, and
the specification step includes calculating a plurality of the evaluation values for specific pixels in the plurality of images, and determining whether the specific pixels are outlier pixels on the basis of the plurality of evaluation values.

6. The image processing method according to claim 4 or 5, wherein the specification step includes calculating a plurality of the evaluation values for a plurality of pixels included in a pixel group in the image, and determining whether the pixel group includes outlier pixels on the basis of the plurality of evaluation values.

7. The image processing method according to any one of claims 1 to 6, wherein the statistical value is a standard deviation or a variance.

8. An image processing device comprising a processor,
wherein the processor
calculates a relationship between luminance values in an image and a statistical value of the luminance values,
acquires an image to be processed,
specifies an outlier pixel that deviates from a trend of output fluctuation indicated by the statistical value, derived on the basis of a luminance value of each pixel of the image and the relationship, using an evaluation value relating to fluctuation in a luminance value calculated for each pixel of the image, and
processes the image using a filter that reduces fluctuation in luminance values with respect to the outlier pixel in the image.

9. The image processing device according to claim 8, wherein the processor specifies the outlier pixel in accordance with a result of comparing the evaluation value calculated on the basis of the luminance value of each pixel of the image with the statistical value of the luminance values.

10. The image processing device according to claim 9, wherein the processor determines whether a specific pixel in the image is an outlier pixel in accordance with a result of comparing a cumulative value of differences between a luminance value of the specific pixel and luminance values of pixels surrounding the specific pixel with a statistical value of the luminance value of the specific pixel.

11. The image processing device according to claim 8, wherein the processor calculates the evaluation value relating to probability from the luminance value of each pixel of the image and the statistical value of the luminance value.

12. The image processing device according to claim 11, wherein the processor
acquires a plurality of the images consecutively over time, and
calculates a plurality of the evaluation values for specific pixels in the plurality of images, and determines whether the specific pixels are outlier pixels on the basis of the plurality of evaluation values.

13. The image processing device according to claim 11 or 12, wherein the processor calculates a plurality of the evaluation values for a plurality of pixels included in a pixel group in the image, and determines whether the pixel group includes outlier pixels on the basis of the plurality of evaluation values.

14. The image processing device according to any one of claims 8 to 13, wherein the statistical value is a standard deviation or a variance.

15. The image processing device according to any one of claims 8 to 14, further comprising a detector configured to detect the image with respect to a subject.

16. An image processing program causing a processor to perform:
a calculation step of calculating a relationship between luminance values in an image and a statistical value of the luminance values;
an acquisition step of acquiring an image to be processed;
a specification step of specifying an outlier pixel that deviates from a trend of output fluctuation indicated by the statistical value, derived on the basis of a luminance value of each pixel of the image and the relationship, using an evaluation value relating to fluctuation in a luminance value calculated for each pixel of the image; and
a processing step of processing the image using a filter that reduces fluctuation in luminance values with respect to the outlier pixel in the image.
